# EUROPEAN PATENT SPECIFICATION

(11) **EP 2 344 569 B1**
(45) Date of publication and mention of the grant of the patent: **13.03.2019**
(21) Application number: 09793166.1
(22) Date of filing: 30.09.2009
(51) Int. Cl.: C08G 77/448, C08L 83/10, A61K 49/04

(54) **X-RAY AND/OR METAL DETECTABLE ARTICLES AND METHOD OF MAKING THE SAME**
RÖNTGEN- UND/ODER METALLDETEKTIERBARE GEGENSTÄNDE UND HERSTELLUNGSVERFAHREN DAFÜR
ARTICLES DÉTECTABLES PAR RAYONS X OU PAR DÉTECTEUR DE MÉTAUX ET LEURS PROCÉDÉS DE FABRICATION

(30) Priority: 30.09.2008 US 242076; 17.02.2009 US 153140 P
(43) Date of publication of application: 20.07.2011
(73) Proprietor: SABIC Global Technologies B.V., 4612 PX Bergen op Zoom (NL)
(72) Inventor: PEEK, Constant, NL-4133 AJ Vianen (NL); VAN DE GRAMPEL, Robert, Dirk, NL-4691 DK Tholen (NL); LENS, Jan-Pleun, NL-3043 BL Rotterdam (NL); PAI-PARANJAPE, Vandita, Evansville IN 47712 (US); HEIN, Christopher, L., Evansville IN 47712 (US)
(74) Representative: Delorme, Nicolas
(86) International application number: PCT/US2009/058977
(87) International publication number: WO 2010/039799

(56) References cited:
- US-A1- 2006 177 379
- US-A1- 2007 048 527
- US-A1- 2007 129 492

## Description

### BACKGROUND OF THE INVENTION

The present invention is directed to thermoplastic compositions having improved metal detectability, and in particular thermoplastic compositions including polycarbonate, a polysiloxane-polycarbonate polymer and an X-ray contrast agents or a metal detectable agent, methods of making and of improving x-ray and/or metal detectability in thermoplastics, and articles prepared therefrom.

Polycarbonate molds are commonly used in food production, such as chocolate molds. Such molds are generally made by plastic injection mold-making which involves high pressure injection of a polycarbonate resin around a metal master. Such polycarbonate molds have a long useful life and are very strong, and are desirable for both ease of use and for making high gloss chocolates. Other types of molds such as those made of silicone rubber or thermoformed plastic are also used for a limited set of applications.

Metal detectors are commonly used in the manufacturing process to detect metallic contaminants that typically arise from wear and tear of machines resulting from routine use, to ensure that food, such as chocolate, prepared from them are of the highest quality. However, metal detectors are not capable of detecting any contaminants coming from plastic components (such as a chocolate mold) in the manufacturing process. X-ray detectors can be used to detect plastics; however, polycarbonate and typical compositions, such as for example those useful for preparing chocolate molds, are transparent to X-rays. Elements having higher atomic numbers than carbon have to be introduced into polycarbonate, either in the form of additives (e.g., glass, pigments, etc.) or incorporated into the polymer chain, to enable articles prepared from the polycarbonate to show any level of X-ray contrast. Typically addition of glass or other inorganic fillers leads to loss of gloss and low transparency as well as reduction in flow and impact properties of polycarbonate. This is not acceptable in those matrices where low temperature ductility in combination with good flow is important.

Document 1 (US 2007/048527) discloses articles comprising a first thermoplastic composition comprising 50 wt% to 70 wt% of PC, 0.5 wt% to 25 wt% polycarbonate-polysiloxane copolymer, 0.5wt% to 20 wt% impact modifier, and 2 wt% to 15 wt% polyetherimide. In particular, document 1 discloses an article comprising a first thermoplastic composition comprising around 70 wt% of polycarbonate and 15 wt% of polycarbonate-polysiloxane copolymer.

Document 2 (US 2006/0177379) discloses combinations of materials for implantable medical devices, including a signal generating agent such as magnetite.

### BRIEF SUMMARY OF THE INVENTION

The present invention provides an article according to claim 1 and a method for increasing the metal detectability in an article according to claim 8.

In one embodiment, the present invention provides an article comprising a thermoplastic composition that includes 65 to 95 parts by weight of polycarbonate, 5 to 35 parts by weight of a polysiloxane-polycarbonate and 0.01 to 10 parts by weight of a metal detectable agent comprising magnetite; wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04.

In another embodiment, the present invention provides method for increasing the metal detectability in an article including a thermoplastic composition, and including the steps of combining a metal detectable agent with polycarbonate and a polysiloxane-polycarbonate, copolymer, the metal detectable agent comprising magnetite, and forming the article from the thermoplastic composition; wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04.

In yet another embodiment, the present invention provides a mold for manufacturing a food product that includes a thermoplastic composition having 65 to 95 parts by weight of polycarbonate, 5 to 35 parts by weight of a polysiloxane-polycarbonate and 0.01 to 10 parts by weight of a metal detectable agent, comprising magnetite; wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04.

A description of the figures, which are meant to be exemplary and not limiting, is provided below.

### BRIEF DESCRIPTION OF THE FIGURES

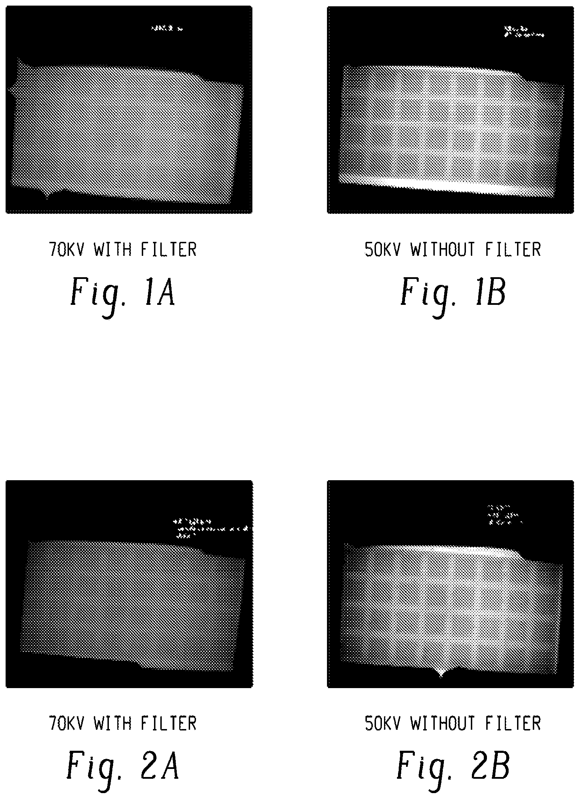

FIG. 1 is an X-ray image of a chocolate bar taken at (A) 70 kV using a filter, and (B) 50 kV taken without a filter.
FIG. 2 is an X-ray image of a chocolate bar with pellets of an exemplary thermoplastic composition having X-ray contrast, scattered over the surface of the chocolate bar, taken at (A) 70 kV using a filter, and (B) 50 kV taken without a filter.

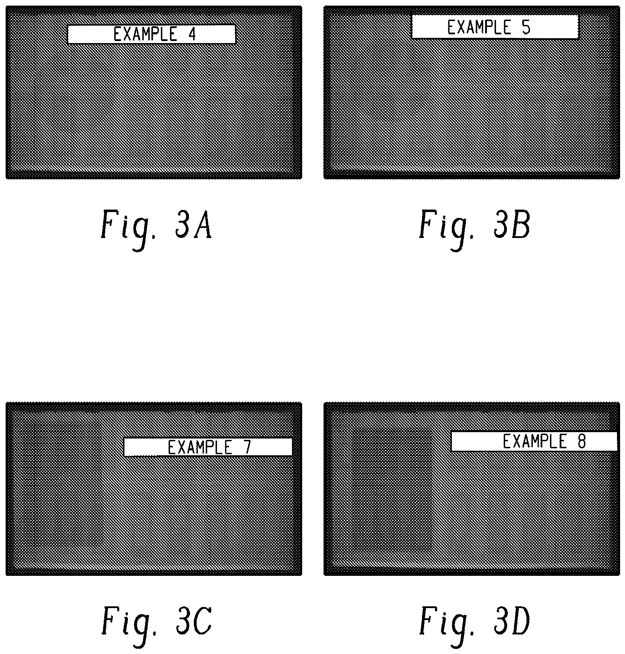

FIG. 3 shows X-ray images of a chocolate bar and an article (circular in A and B, oblong in C and D) placed on the chocolate bar, in which the articles are molded from the composition of (A) Example 4, (B) Example 5, (C) Example 7, and (D) Example 8.
FIG. 4 shows a plot of ejection pressure versus cycle time to demonstrate mold release properties for articles molded from a polycarbonate control, Example 9, and Comparative Examples 13 and 14.
FIGS. 5 and 6 are graphs showing impact strength and impact retention of molded articles made according to the metal-detectable concepts of the present invention as compared to prior art compositions and showing 0, 3.5 and 5 total wt.% Si.
FIG. 7 is a graph showing fatigue data for metal detectable compositions of the present invention as compared to prior art compositions, again showing 0, 3.5 and 5 total wt.% Si.

The above described and other features are exemplified by the following detailed description.

### DETAILED DESCRIPTION OF THE INVENTION

This disclosure provides a thermoplastic composition that is detectable by a metal detector and is prepared from polycarbonate, a polysiloxane-polycarbonate copolymer and a metal detectable agent. Articles made from the thermoplastic composition are also disclosed.

It has been found that certain compounds are useful as metal detectable agents for formulating the metal detectable thermoplastic composition. Specifically useful compounds include ferrous metals and nonferrous metals, such as magnetite, selected from within an optimal particle size range. These compounds are useful in formulating such metal detectable compositions having the aforementioned properties. Common inorganic compounds useful as metal detectable agents are ferrous metals (having both good magnetic permeability and electrical conductivity). The particle size of the metal detectable agent is selected such that select properties (flow, low temperature impact, gloss) of the composition are maintained.

The thermoplastic compositions used in the present invention include both a polycarbonate and a polysiloxane-polycarbonate copolymer, also referred to as a polysiloxane-polycarbonate. As used herein, the terms "polycarbonate" and "polycarbonate resin" mean compositions having repeating structural carbonate units of the formula (1): 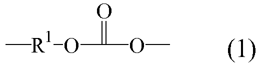 in which at least 60 percent of the total number of R¹ groups are aromatic organic radicals and the balance thereof are aliphatic, alicyclic, or aromatic radicals. In one embodiment, each R¹ is an aromatic organic radical, for example a radical of the formula (2):

-A¹-Y¹-A²- (2)

wherein each of A¹ and A² is a monocyclic divalent aryl radical and Y¹ is a bridging radical having one or two atoms that separate A¹ from A². In an exemplary embodiment, one atom separates A¹ from A². Illustrative non-limiting examples of radicals of this type are -O-, -S-, -S(O)-, -S(O)₂-, -C(O)-, methylene, cyclohexyl-methylene, 2-[2.2.1]-bicycloheptylidene, ethylidene, isopropylidene, neopentylidene, cyclohexylidene, cyclopentadecylidene, cyclododecylidene, and adamantylidene. The bridging radical Y¹ may be a hydrocarbon group or a saturated hydrocarbon group such as methylene, cyclohexylidene, or isopropylidene.

Polycarbonates may be produced by the reaction of dihydroxy compounds having the formula HO-R¹-OH, which includes dihydroxy compounds of formula (3):

HO-A¹-Y¹-A²-OH (3)

wherein Y¹, A¹ and A² are as described above. Also included are bisphenol compounds of general formula (4): 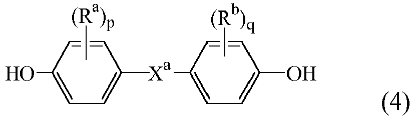 wherein R^{a} and R^{b} each represent a halogen atom or a monovalent hydrocarbon group and may be the same or different; p and q are each independently integers of 0 to 4; and X^{a} represents one of the groups of formula (5): 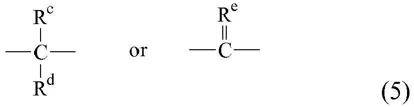 wherein R^{c} and R^{d} each independently represent a hydrogen atom or a monovalent linear or cyclic hydrocarbon group and R^{e} is a divalent hydrocarbon group.

In an embodiment, a heteroatom-containing cyclic alkylidene group includes at least one heteroatom with a valency of 2 or greater, and at least two carbon atoms. Heteroatoms for use in the heteroatom-containing cyclic alkylidene group include -O-, -S-, and -N(Z)-, where Z is a substituent group selected from hydrogen, hydroxy, C₁₋₁₂ alkyl, C₁₋₁₂ alkoxy, or C₁₋₁₂ acyl. Where present, the cyclic alkylidene group or heteroatom-containing cyclic alkylidene group may have 3 to 20 atoms, and may be a single saturated or unsaturated ring, or fused polycyclic ring system wherein the fused rings are saturated, unsaturated, or aromatic.

Other bisphenols containing substituted or unsubstituted cyclohexane units can be used, for example bisphenols of formula (6): 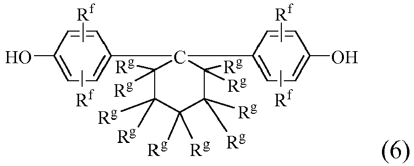 wherein each R^{f} is independently hydrogen, C₁₋₁₂ alkyl, or halogen; and each R^{g} is independently hydrogen or C₁₋₁₂ alkyl. The substituents may be aliphatic or aromatic, straight chain, cyclic, bicyclic, branched, saturated, or unsaturated. Such cyclohexane-containing bisphenols, for example the reaction product of two moles of a phenol with one mole of a hydrogenated isophorone, are useful for making polycarbonate polymers with high glass transition temperatures and high heat distortion temperatures. Cyclohexyl bisphenol containing polycarbonates, or a combination including at least one of the foregoing with other bisphenol polycarbonates, are supplied by Bayer Co. under the APEC^{®} trade name.

Other useful dihydroxy compounds having the formula HO-R¹-OH include aromatic dihydroxy compounds of formula (7): 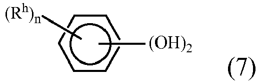 wherein each R^{h} is independently a halogen atom, a C₁₋₁₀ hydrocarbyl such as a C₁₋₁₀ alkyl group, a halogen substituted C₁₋₁₀ hydrocarbyl such as a halogen-substituted C₁₋₁₀ alkyl group, and n is 0 to 4. The halogen is usually bromine.

Exemplary dihydroxy compounds include the following: 4,4'-dihydroxybiphenyl, 1,6-dihydroxynaphthalene, 2,6-dihydroxynaphthalene, bis(4-hydroxyphenyl)methane, bis(4-hydroxyphenyl)diphenylmethane, bis(4-hydroxyphenyl)-1-naphthylmethane, 1,2-bis(4-hydroxyphenyl)ethane, 1,1-bis(4-hydroxyphenyl)-1-phenylethane, 2-(4-hydroxyphenyl)-2-(3-hydroxyphenyl)propane, bis(4-hydroxyphenyl)phenylmethane, 2,2-bis(4-hydroxy-3-bromophenyl)propane, 1,1-bis (hydroxyphenyl)cyclopentane, 1,1-bis(4-hydroxyphenyl)cyclohexane, 1,1-bis(4-hydroxyphenyl)isobutene, 1,1-bis(4-hydroxyphenyl)cyclododecane, trans-2,3-bis(4-hydroxyphenyl)-2-butene, 2,2-bis(4-hydroxyphenyl)adamantine, (alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-t-butyl-4-hydroxyphenyl)propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl)hexafluoropropane, 1,1-dichloro-2,2-bis(4-hydroxyphenyl)ethylene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl)ether, bis(4-hydroxyphenyl)ether, bis(4-hydroxyphenyl)sulfide, bis(4-hydroxyphenyl)sulfoxide, bis(4-hydroxyphenyl)sulfone, 9,9-bis(4-hydroxyphenyl)fluorine, 2,7-dihydroxypyrene, 6,6'-dihydroxy-3,3,3',3'- tetramethylspiro(bis)indane ("spirobiindane bisphenol"), 3,3-bis(4-hydroxyphenyl)phthalide, 2,6-dihydroxydibenzo-p-dioxin, 2,6-dihydroxythianthrene, 2,7-dihydroxyphenoxathin, 2,7-dihydroxy-9,10-dimethylphenazine, 3,6-dihydroxydibenzofuran, 3,6-dihydroxydibenzothiophene, and 2,7-dihydroxycarbazole, resorcinol, substituted resorcinol compounds such as 5-methyl resorcinol, 5-ethyl resorcinol, 5-propyl resorcinol, 5-butyl resorcinol, 5-t-butyl resorcinol, 5-phenyl resorcinol, 5-cumyl resorcinol, 2,4,5,6-tetrafluoro resorcinol, 2,4,5,6-tetrabromo resorcinol, or the like; catechol; hydroquinone; substituted hydroquinones such as 2-methyl hydroquinone, 2-ethyl hydroquinone, 2-propyl hydroquinone, 2-butyl hydroquinone, 2-t-butyl hydroquinone, 2-phenyl hydroquinone, 2-cumyl hydroquinone, 2,3,5,6-tetramethyl hydroquinone, 2,3,5,6-tetra-t-butyl hydroquinone, 2,3,5,6-tetrafluoro hydroquinone, 2,3,5,6-tetrabromo hydroquinone, and the like, as well as combinations including at least one of the foregoing dihydroxy compounds.

Specific examples of bisphenol compounds that may be represented by formula (3) include 1,1-bis(4-hydroxyphenyl) methane, 1,1-bis(4-hydroxyphenyl) ethane, 2,2-bis(4-hydroxyphenyl) propane (hereinafter "bisphenol A" or "BPA"), 2,2-bis(4-hydroxyphenyl) butane, 2,2-bis(4-hydroxyphenyl) octane, 1,1-bis(4-hydroxyphenyl) propane, 1,1-bis(4-hydroxyphenyl) n-butane, 2,2-bis(4-hydroxy-1-methylphenyl) propane, 1,1-bis(4-hydroxy-t-butylphenyl) propane, 3,3-bis(4-hydroxyphenyl) phthalimidine, 2-phenyl-3,3-bis(4-hydroxyphenyl) phthalimidine (PPPBP), and 1,1-bis(4-hydroxy-3-methylphenyl)cyclohexane (DMBPC). Combinations including at least one of the foregoing dihydroxy compounds may also be used.

In a specific embodiment, the polycarbonate may be a linear homopolymer derived from bisphenol A, in which each of A¹ and A² is p-phenylene and Y¹ is isopropylidene. The polycarbonates may have an intrinsic viscosity, as determined in chloroform at 25°C, of 0.3 to 1.5 deciliters per gram (dl/g), specifically 0.45 to 1.0 dl/g. The polycarbonates may have a weight average molecular weight (Mw) of 10,000 to 100,000 g/mol, as measured by gel permeation chromatography (GPC) using a crosslinked styrene-divinyl benzene column, at a sample concentration of 1 milligram per milliliter, and as calibrated with polycarbonate standards.

In an embodiment, the polycarbonate may have a melt volume flow rate (often abbreviated MVR) measures the rate of extrusion of a thermoplastics through an orifice at a prescribed temperature and load. Polycarbonates useful for the formation of articles may have an MVR, measured at 300°C under a load of 1.2 kg according to ASTM D1238-04 or ISO 1133, of 0.5 to 80 cubic centimeters per 10 minutes (cc/10 min). In a specific embodiment, a useful polycarbonate or combination of polycarbonates (i.e., a polycarbonate composition) has an MVR measured at 300°C under a load of 1.2 kg according to ASTM D1238-04 or ISO 1133, of 0.5 to 20 cc/10 min, specifically 0.5 to 18 cc/10 min, and more specifically 1 to 15 cc/10 min.

"Polycarbonates" and "polycarbonate resins" as used herein further include homopolycarbonates, copolymers including different R¹ moieties in the carbonate (referred to herein as "copolycarbonates"), copolymers including carbonate units and other types of polymer units, such as ester units, polysiloxane units, and combinations including at least one of homopolycarbonates and copolycarbonates. As used herein, "combination" is inclusive of blends, mixtures, alloys, reaction products, and the like. A specific type of copolymer is a polyester carbonate, also known as a polyester-polycarbonate. Such copolymers further contain, in addition to recurring carbonate chain units of the formula (1), repeating units of formula (8): 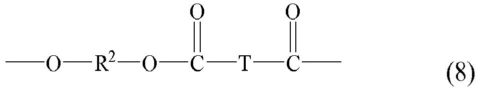 wherein R² is a divalent group derived from a dihydroxy compound, and may be, for example, a C₂₋₁₀ alkylene group, a C₆₋₂₀ alicyclic group, a C₆₋₂₀ aromatic group or a polyoxyalkylene group in which the alkylene groups contain 2 to 6 carbon atoms, specifically 2, 3, or 4 carbon atoms; and T divalent group derived from a dicarboxylic acid, and may be, for example, a C₂₋₁₀ alkylene group, a C₆₋₂₀ alicyclic group, a C₆₋₂₀ alkyl aromatic group, or a C₆₋₂₀ aromatic group.

In an embodiment, R² is a C₂₋₃₀ alkylene group having a straight chain, branched chain, or cyclic (including polycyclic) structure. In another embodiment, R² is derived from an aromatic dihydroxy compound of formula (4) above. In another embodiment, R² is derived from an aromatic dihydroxy compound of formula (7) above.

Examples of aromatic dicarboxylic acids that may be used to prepare the polyester units include isophthalic or terephthalic acid, 1,2-di(p-carboxyphenyl)ethane, 4,4'-dicarboxydiphenyl ether, 4,4'-bisbenzoic acid, and combinations including at least one of the foregoing acids. Acids containing fused rings can also be present, such as in 1,4-, 1,5-, or 2,6-naphthalenedicarboxylic acids. Specific dicarboxylic acids are terephthalic acid, isophthalic acid, naphthalene dicarboxylic acid, cyclohexane dicarboxylic acid, or combinations thereof. A specific dicarboxylic acid includes a combination of isophthalic acid and terephthalic acid wherein the weight ratio of isophthalic acid to terephthalic acid is 91:9 to 2:98. In another specific embodiment, R² is a C₂₋₆ alkylene group and T is p-phenylene, m-phenylene, naphthalene, a divalent cycloaliphatic group, or a combination thereof. This class of polyester includes the poly(alkylene terephthalates).

The molar ratio of ester units to carbonate units in the copolymers may vary broadly, for example 1:99 to 99:1, specifically 10:90 to 90:10, more specifically 25:75 to 75:25, depending on the selected properties of the final composition.

Processes such as interfacial polymerization and melt polymerization can be used manufacture polycarbonates. Although the reaction conditions for interfacial polymerization may vary, an exemplary process generally involves dissolving or dispersing a dihydric phenol reactant in aqueous caustic soda or potash, adding the resulting mixture to a suitable water-immiscible solvent medium, and contacting the reactants with a carbonate precursor in the presence of a catalyst such as triethylamine or a phase transfer catalyst, under controlled pH conditions, e.g., 8 to 10. The most commonly used water immiscible solvents include methylene chloride, 1,2-dichloroethane, chlorobenzene, toluene, and the like.

Carbonate precursors include, for example, a carbonyl halide such as carbonyl bromide or carbonyl chloride, or a haloformate such as a bishaloformates of a dihydric phenol (e.g., the bischloroformates of bisphenol A, hydroquinone, or the like) or a glycol (e.g., the bishaloformate of ethylene glycol, neopentyl glycol, polyethylene glycol, or the like). Combinations including at least one of the foregoing types of carbonate precursors may also be used. In an exemplary embodiment, an interfacial polymerization reaction to form carbonate linkages uses phosgene as a carbonate precursor, and is referred to as a phosgenation reaction.

Among the phase transfer catalysts that may be used are catalysts of the formula (R³)₄Q⁺X, wherein each R³ is the same or different, and is a C₁₋₁₀ alkyl group; Q is a nitrogen or phosphorus atom; and X is a halogen atom or a C₁₋₈ alkoxy group or C₆₋₁₈ aryloxy group. Useful phase transfer catalysts include, for example, [CH₃(CH₂)₃]₄NX, [CH₃(CH₂)₃]₄PX, [CH₃(CH₂)₅]₄NX, [CH₃(CH₂)₆]₄NX, [CH₃(CH₂)₄]₄NX, CH₃[CH₃(CH₂)₃]₃NX, and CH_{3[}CH₃(CH₂)_{2]3}NX, wherein X is Cl⁻, Br⁻, a C₁₋₈ alkoxy group or a C₆₋₁₈ aryloxy group. An effective amount of a phase transfer catalyst may be 0.1 to 10 wt% based on the weight of bisphenol in the phosgenation mixture. In another embodiment an effective amount of phase transfer catalyst may be 0.5 to 2 wt% based on the weight of bisphenol in the phosgenation mixture.

All types of polycarbonate end groups are contemplated as being useful in the polycarbonate composition, provided that such end groups do not significantly adversely affect selected properties of the compositions.

Branched polycarbonate blocks may be prepared by adding a branching agent during polymerization. These branching agents include polyfunctional organic compounds containing at least three functional groups selected from hydroxyl, carboxyl, carboxylic anhydride, haloformyl, and mixtures of the foregoing functional groups. Specific examples include trimellitic acid, trimellitic anhydride, trimellitic trichloride, tris-p-hydroxy phenyl ethane, isatin-bis-phenol, tris-phenol TC (1,3,5-tris((p-hydroxyphenyl)isopropyl)benzene), tris-phenol PA (4(4(1,1-bis(p-hydroxyphenyl)-ethyl) alpha, alpha-dimethyl benzyl)phenol), 4-chloroformyl phthalic anhydride, trimesic acid, and benzophenone tetracarboxylic acid. The branching agents may be added at a level of 0.05 to 2.0 wt%. Mixtures including linear polycarbonates and branched polycarbonates may be used.

A chain stopper (also referred to as a capping agent) may be included during polymerization. The chain stopper limits molecular weight growth rate, and so controls molecular weight in the polycarbonate. Exemplary chain stoppers include certain mono-phenolic compounds, mono-carboxylic acid chlorides, and/or mono-chloroformates. Mono-phenolic chain stoppers are exemplified by monocyclic phenols such as phenol and C₁-C₂₂ alkyl-substituted phenols such as p-cumyl-phenol, resorcinol monobenzoate, and p-and tertiary-butyl phenol; and monoethers of diphenols, such as p-methoxyphenol. Alkyl-substituted phenols with branched chain alkyl substituents having 8 to 9 carbon atom may be specifically mentioned. Certain mono-phenolic UV absorbers may also be used as a capping agent, for example 4-substituted-2-hydroxybenzophenones and their derivatives, aryl salicylates, monoesters of diphenols such as resorcinol monobenzoate, 2-(2-hydroxyaryl)-benzotriazoles and their derivatives, 2-(2-hydroxyaryl)-1,3,5-triazines and their derivatives, and the like.

Mono-carboxylic acid chlorides may also be used as chain stoppers. These include monocyclic, mono-carboxylic acid chlorides such as benzoyl chloride, C₁-C₂₂ alkyl-substituted benzoyl chloride, toluoyl chloride, halogen-substituted benzoyl chloride, bromobenzoyl chloride, cinnamoyl chloride, 4-nadimidobenzoyl chloride, and combinations thereof; polycyclic, mono-carboxylic acid chlorides such as trimellitic anhydride chloride, and naphthoyl chloride; and combinations of monocyclic and polycyclic mono-carboxylic acid chlorides. Chlorides of aliphatic monocarboxylic acids with less than or equal to 22 carbon atoms are useful. Functionalized chlorides of aliphatic monocarboxylic acids, such as acryloyl chloride and methacryoyl chloride, are also useful. Also useful are mono-chloroformates including monocyclic, mono-chloroformates, such as phenyl chloroformate, alkyl-substituted phenyl chloroformate, p-cumyl phenyl chloroformate, toluene chloroformate, and combinations thereof.

Alternatively, melt processes may be used to make the polycarbonates. Generally, in the melt polymerization process, polycarbonates may be prepared by co-reacting, in a molten state, the dihydroxy reactant(s) and a diaryl carbonate ester, such as diphenyl carbonate, in the presence of a transesterification catalyst in a BANBURY® mixer, twin screw extruder, or the like to form a uniform dispersion. Volatile monohydric phenol is removed from the molten reactants by distillation and the polymer is isolated as a molten residue. A specifically useful melt process for making polycarbonates uses a diaryl carbonate ester having electron-withdrawing substituents on the aryls. Examples of specifically useful diaryl carbonate esters with electron withdrawing substituents include bis(4-nitrophenyl)carbonate, bis(2-chlorophenyl)carbonate, bis(4-chlorophenyl)carbonate, bis(methyl salicyl)carbonate, bis(4-methylcarboxylphenyl) carbonate, bis(2-acetylphenyl) carboxylate, bis(4-acetylphenyl) carboxylate, or a combination including at least one of the foregoing. In addition, transesterification catalysts for use may include phase transfer catalysts of formula (R³)₄Q⁺X above, wherein each R³, Q, and X are as defined above. Examples of transesterification catalysts include tetrabutylammonium hydroxide, methyltributylammonium hydroxide, tetrabutylammonium acetate, tetrabutylphosphonium hydroxide, tetrabutylphosphonium acetate, tetrabutylphosphonium phenolate, or a combination including at least one of the foregoing.

In addition to the polycarbonates described above, combinations of the polycarbonate with other thermoplastic polymers, for example combinations of homopolycarbonates and/or polycarbonate copolymers with polyesters, may be used. Useful polyesters may include, for example, polyesters having repeating units of formula (8), which include poly(alkylene dicarboxylates), liquid crystalline polyesters, and polyester copolymers. The polyesters described herein are generally completely miscible with the polycarbonates when blended.

The polyesters may be obtained by interfacial polymerization or melt-process condensation as described above, by solution phase condensation, or by transesterification polymerization wherein, for example, a dialkyl ester such as dimethyl terephthalate may be transesterified with ethylene glycol using acid catalysis, to generate poly(ethylene terephthalate). It is possible to use a branched polyester in which a branching agent, for example, a glycol having three or more hydroxyl groups or a trifunctional or multifunctional carboxylic acid has been incorporated. Furthermore, it is sometime desirable to have various concentrations of acid and hydroxyl end groups on the polyester, depending on the ultimate end use of the composition.

The polyester-polycarbonates may also be prepared by interfacial polymerization. Rather than utilizing the dicarboxylic acid per se, it is possible, and sometimes even preferred, to employ the reactive derivatives of the acid, such as the corresponding acid halides, in particular the acid dichlorides and the acid dibromides. Thus, for example instead of using isophthalic acid, terephthalic acid, or a combination comprising at least one of the foregoing, it is possible to employ isophthaloyl dichloride, terephthaloyl dichloride, and a combination comprising at least one of the foregoing.

The polyesters may be obtained by interfacial polymerization or melt-process condensation as described above, by solution phase condensation, or by transesterification polymerization wherein, for example, a dialkyl ester such as dimethyl terephthalate may be transesterified with ethylene glycol using acid catalysis, to generate poly(ethylene terephthalate). It is possible to use a branched polyester in which a branching agent, for example, a glycol having three or more hydroxyl groups or a trifunctional or multifunctional carboxylic acid has been incorporated. Furthermore, it is sometime desirable to have various concentrations of acid and hydroxyl end groups on the polyester, depending on the ultimate end use of the composition.

Useful polyesters may include aromatic polyesters, poly(alkylene esters) including poly(alkylene arylates), and poly(cycloalkylene diesters). Aromatic polyesters may have a polyester structure according to formula (8), wherein D and T are each aromatic groups as described hereinabove. In an embodiment, useful aromatic polyesters may include, for example, poly(isophthalate-terephthalate-resorcinol) esters, poly(isophthalate-terephthalate-bisphenol-A) esters, poly[(isophthalate-terephthalate-resorcinol) ester-*co-*(isophthalate-terephthalate-bisphenol-A)] ester, or a combination comprising at least one of these. Also contemplated are aromatic polyesters with a minor amount, e.g., 0.5 to 10 wt%, based on the total weight of the polyester, of units derived from an aliphatic diacid and/or an aliphatic polyol to make copolyesters. Poly(alkylene arylates) may have a polyester structure according to formula (8), wherein T comprises groups derived from aromatic dicarboxylates, cycloaliphatic dicarboxylic acids, or derivatives thereof. Examples of specifically useful T groups include 1,2-, 1,3-, and 1,4-phenylene; 1,4- and 1,5- naphthylenes; cis- or trans-1,4-cyclohexylene; and the like. Specifically, where T is 1,4-phenylene, the poly(alkylene arylate) is a poly(alkylene terephthalate). In addition, for poly(alkylene arylate), specifically useful alkylene groups D include, for example, ethylene, 1,4-butylene, and bis-(alkylene-disubstituted cyclohexane) including cis- and/or trans-1,4-(cyclohexylene)dimethylene. Examples of poly(alkylene terephthalates) include poly(ethylene terephthalate) (PET), poly(1,4-butylene terephthalate) (PBT), and poly(propylene terephthalate) (PPT). Also useful are poly(alkylene naphthoates), such as poly(ethylene naphthanoate) (PEN), and poly(butylene naphthanoate) (PBN). A useful poly(cycloalkylene diester) is poly(cyclohexanedimethylene terephthalate) (PCT). Combinations comprising at least one of the foregoing polyesters may also be used.

Copolymers comprising alkylene terephthalate repeating ester units with other ester groups may also be useful. Useful ester units may include different alkylene terephthalate units, which can be present in the polymer chain as individual units, or as blocks of poly(alkylene terephthalates). Specific examples of such copolymers include poly(cyclohexanedimethylene terephthalate)-co-poly(ethylene terephthalate), abbreviated as PETG where the polymer comprises greater than or equal to 50 mol% of poly(ethylene terephthalate), and abbreviated as PCTG where the polymer comprises greater than 50 mol% of poly(1,4-cyclohexanedimethylene terephthalate).

Poly(cycloalkylene diester)s may also include poly(alkylene cyclohexanedicarboxylate)s. Of these, a specific example is poly(1,4-cyclohexanedimethanol-1,4-cyclohexanedicarboxylate) (PCCD), having recurring units of formula (9): 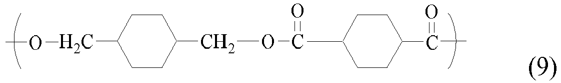 wherein, as described using formula (8), R² is a 1,4-cyclohexanedimethylene group derived from 1,4-cyclohexanedimethanol, and T is a cyclohexane ring derived from cyclohexanedicarboxylate or a chemical equivalent thereof, and may comprise the cis-isomer, the trans-isomer, or a combination comprising at least one of the foregoing isomers.

The polycarbonate and polyester and/or polyester-polycarbonate may be used in a weight ratio of 1:99 to 99:1, specifically 10:90 to 90:10, and more specifically 30:70 to 70:30, depending on the function and properties desired.

The polyester-polycarbonates may have a weight average molecular weight (M_{w}) of 1,500 to 100,000 g/mol, specifically 1,700 to 50,000 g/mol, more specifically 2,000 to 40,000 g/mol, and still more specifically 5,000 to 30,000 g/mol. Molecular weight determinations are performed using gel permeation chromatography (GPC), using a crosslinked styrene-divinylbenzene column and calibrated to polycarbonate references. Samples are prepared at a concentration of 1 mg/ml, and are eluted at a flow rate of 1.0 ml/min.

Where used, it is desirable for a polyester-polycarbonate to have an MVR of 5 to 150 cc/10 min., specifically 7 to 125 cc/10 min, more specifically 9 to 110 cc/10 min, and still more specifically 10 to 100 cc/10 min., measured at 300°C and a load of 1.2 kilograms according to ASTM D1238-04. Commercial polyester blends with polycarbonate are marketed under the trade name XYLEX®, including for example XYLEX® X7300, and commercial polyester-polycarbonates are marketed under the tradename LEXAN™ SLX polymers, including for example LEXAN™ SLX-9000, and are available from SABIC Innovative Plastics (formerly GE Plastics).

In addition to the polycarbonate, the thermoplastic compositions used in the present invention include a polysiloxane-polycarbonate copolymer that also includes a polycarbonate with a polysiloxane. The polysiloxane (also referred to herein as "polydiorganosiloxane") blocks of the copolymer include repeating siloxane units (also referred to herein as "diorganosiloxane units") of formula (10): 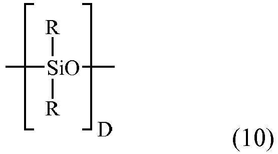 wherein each occurrence of R is same or different, and is a C₁₋₁₃ monovalent organic radical. For example, R may independently be a C₁-C₁₃ alkyl group, C₁-C₁₃ alkoxy group, C₂-C₁₃ alkenyl group, C₂-C₁₃ alkenyloxy group, C₃-C₆ cycloalkyl group, C₃-C₆ cycloalkoxy group, C6-C14 aryl group, C₆-C₁₀ aryloxy group, C₇-C₁₃ arylalkyl group, C₇-C₁₃ arylalkoxy group, C₇-C₁₃ alkylaryl group, or C₇-C₁₃ alkylaryloxy group. The foregoing groups may be fully or partially halogenated with fluorine, chlorine, bromine, or iodine, or a combination thereof. Combinations of the foregoing R groups may be used in the same copolymer.

The value of D in formula (10) may vary widely depending on the type and relative amount of each component in the thermoplastic composition, the selected properties of the composition, and like considerations. Generally, D may have an average value of 2 to 1,000, specifically 2 to 500, more specifically 5 to 100, and still more specifically 5 to 50. In a specific embodiment, D has an average value of 40 to 50. In an exemplary embodiment, D has an average value of 45. In another specific embodiment, D has an average value of 20 to 40. In another exemplary embodiment, D has an average value of 30.

Where D is of a lower value, e.g., less than 40, it may be beneficial to use a relatively larger amount of the polycarbonate-polysiloxane copolymer. Conversely, where D is of a higher value, e.g., greater than 40, it may be necessary to use a relatively lower amount of the polycarbonate-polysiloxane copolymer.

A combination of a first and a second (or more) polysiloxane-polycarbonate copolymer may be used, wherein the average value of D of the first copolymer is less than the average value of D of the second copolymer.

In one embodiment, the polydiorganosiloxane blocks are provided by repeating structural units of formula (11): 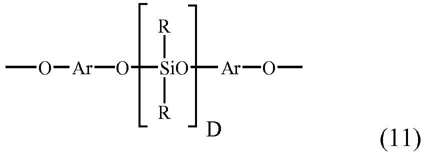 wherein D is as defined above; each R may independently be the same or different, and is as defined above; and each Ar may independently be the same or different, and is a substituted or unsubstituted C₆-C₃₀ arylene radical, wherein the bonds are directly connected to an aromatic moiety. Useful Ar groups in formula (11) may be derived from a C₆-C₃₀ dihydroxyarylene compound, for example a dihydroxyarylene compound of formula (3), (4), or (7) above. Combinations including at least one of the foregoing dihydroxyarylene compounds may also be used. Specific examples of dihydroxyarylene compounds are 1,1-bis(4-hydroxyphenyl) methane, 1,1-bis(4-hydroxyphenyl) ethane, 2,2-bis(4-hydroxyphenyl) propane, 2,2-bis(4-hydroxyphenyl) butane, 2,2-bis(4-hydroxyphenyl) octane, 1,1-bis(4-hydroxyphenyl) propane, 1,1-bis(4-hydroxyphenyl) n-butane, 2,2-bis(4-hydroxy-1-methylphenyl) propane, 1,1-bis(4-hydroxyphenyl) cyclohexane, bis(4-hydroxyphenyl sulphide), and 1,1-bis(4-hydroxy-t-butylphenyl) propane. Combinations including at least one of the foregoing dihydroxy compounds may also be used.

Units of formula (11) may be derived from the corresponding dihydroxy compound of formula (12): 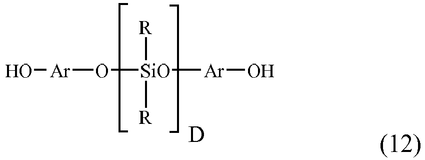 wherein R, Ar, and D are as described above. Compounds of formula (12) may be obtained by the reaction of a dihydroxyarylene compound with, for example, an alpha, omega-bisacetoxypolydiorangonosiloxane under phase transfer conditions.

In another embodiment, polydiorganosiloxane blocks include units of formula (13): 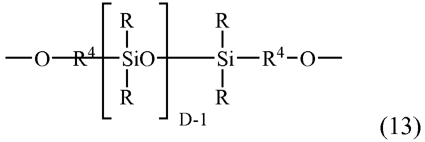 wherein R and D are as described above, and each occurrence of R⁴ is independently a divalent C₁-C₃₀ alkylene, and wherein the polymerized polysiloxane unit is the reaction residue of its corresponding dihydroxy compound. In a specific embodiment, the polydiorganosiloxane blocks are provided by repeating structural units of formula (14): 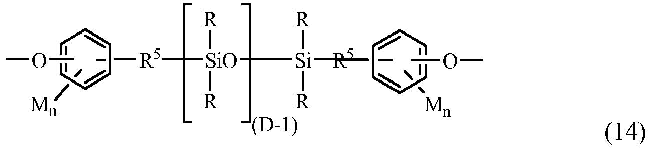 wherein R and D are as defined above. Each R⁵ in formula (14) is independently a divalent C₂-C₈ aliphatic group. Each M in formula (13) may be the same or different, and may be a halogen, cyano, nitro, C₁-C₈ alkylthio, C₁-C₈ alkyl, C₁-C₈ alkoxy, C₂-C₈ alkenyl, C₂-C₈ alkenyloxy group, C₃-C₈ cycloalkyl, C₃-C₈ cycloalkoxy, C₆-C₁₀ aryl, C₆-C₁₀ aryloxy, C₇-C₁₂ arylalkyl, C₇-C₁₂ arylalkoxy, C₇-C₁₂ alkylaryl, or C₇-C₁₂ alkylaryloxy, wherein each n is independently 0, 1, 2, 3, or 4.

In one embodiment, M is bromo or chloro, an alkyl group such as methyl, ethyl, or propyl, an alkoxy group such as methoxy, ethoxy, or propoxy, or an aryl group such as phenyl, chlorophenyl, or tolyl; R⁵ is a dimethylene, trimethylene or tetramethylene group; and R is a C₁₋₈ alkyl, haloalkyl such as trifluoropropyl, cyanoalkyl, or aryl such as phenyl, chlorophenyl or tolyl. In another embodiment, R is methyl, or a mixture of methyl and trifluoropropyl, or a mixture of methyl and phenyl. In still another embodiment, M is methoxy, n is one, R⁵ is a divalent C₁-C₃ aliphatic group, and R is methyl.

Units of formula (14) may be derived from the corresponding dihydroxy polydiorganosiloxane (15): 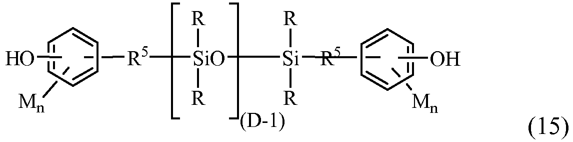 wherein R, D, M, R⁵, and n are as described above. Such dihydroxy polysiloxanes can be made by effecting a platinum catalyzed addition between a siloxane hydride of formula (16): 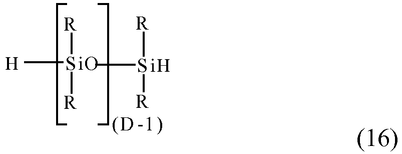 wherein R and D are as previously defined, and an aliphatically unsaturated monohydric phenol. Useful aliphatically unsaturated monohydric phenols included, for example, eugenol, 2-allylphenol, 4-allyl-2-methylphenol, 4-allyl-2-phenylphenol, 4-allyl-2-bromophenol, 4-allyl-2-t-butoxyphenol, 4-phenyl-2-phenylphenol, 2-methyl-4-propylphenol, 2-allyl-4,6-dimethylphenol, 2-allyl-4-bromo-6-methylphenol, 2-allyl-6-methoxy-4-methylphenol and 2-allyl-4,6-dimethylphenol. Mixtures including at least one of the foregoing may also be used.

The polysiloxane-polycarbonate includes 50 to 99.9 wt% of carbonate units and 0.1 to 50 wt% siloxane units, specifically 0.1 to 25 wt% siloxane units, based on the total weight of the polysiloxane-polycarbonate. In a specific embodiment, the polysiloxane-polycarbonate copolymer includes 90 to 99 wt%, more specifically 92 to 98 wt%, still more specifically 93 to 97 wt%, and still more specifically 93 to 96 wt% of carbonate units and 1 to 10 wt%, specifically 2 to 8 wt%, more specifically 3 to 7 wt%, and still more specifically 4 to 7 wt% siloxane units. In an exemplary embodiment the polysiloxane-polycarbonate includes 6 wt% siloxane units. In another specific embodiment, the polysiloxane-polycarbonate copolymer includes 75 to 90 wt%, more specifically 75 to 85 wt%, still more specifically 77 to 83 wt%, and still more specifically 78 to 82 wt% of carbonate units and 10 to 25 wt%, specifically 15 to 25 wt%, more specifically 17 to 23 wt%, and still more specifically 18 to 22 wt% siloxane units. In another exemplary embodiment the polysiloxane-polycarbonate includes 20 wt% siloxane units. All references to weight percent compositions in the polysiloxane-polycarbonate are based on the total weight of the polysiloxane-polycarbonate.

In an embodiment, the polysiloxane-polycarbonate includes polysiloxane units, and carbonate units derived from bisphenol A, e.g., the dihydroxy compound of formula (3) in which each of A¹ and A² is p-phenylene and Y¹ is isopropylidene. Polysiloxane-polycarbonates may have a weight average molecular weight of 2,000 to 100,000 g/mol, specifically 5,000 to 50,000 g/mol as measured by gel permeation chromatography using a crosslinked styrene-divinyl benzene column, at a sample concentration of 1 milligram per milliliter, and as calibrated with polycarbonate standards.

The polysiloxane-polycarbonate can have a melt volume flow rate, measured at 300°C under a load of 1.2 kg, of 1 to 50 cubic centimeters per 10 minutes (cc/10 min), specifically 2 to 30 cc/10 min. Mixtures of polysiloxane-polycarbonates of different flow properties may be used to achieve the overall selected flow property. In an embodiment, exemplary polysiloxane-polycarbonates are marketed under the trade name LEXAN™ EXL polycarbonates, available from SABIC Innovative Plastics (formerly GE Plastics).

The thermoplastic composition includes a metal detectable agent. The metal detectable agent can be any ferrous or nonferrous metal having good magnetic permeability and/or good electrical conductivity. Most standard metal detectors are capable of detecting materials that are magnetic, such as those containing iron, such as magnetite.

In addition, it has been discovered that for compositions having both polycarbonate and a polycarbonate-polysiloxane copolymer, the addition of the metal detectable agent surprisingly helps to retain impact strength even when less siloxane is included in the final composition. In general, the greater the amount of siloxane in the final composition, the better the impact properties. However, in the compositions of the present invention, it has been discovered that better impact properties, such as notched izod impact, can be obtained with compositions having lower amounts of siloxane and a small amount of metal detectable agent.

The metal detectable agent is in the form of particles, and may, depending upon the type used, be a filler, pigment, or other such class of inorganic material having suitable properties as defined herein. The particles as disclosed herein can be used in any suitable morphological form, including but not limited to microparticles, nanoparticles, and particles having various shapes including spheres, rods, faceted crystalline shapes, irregular shapes, and the like. The size of the particles of the metal detectable agent, as measured by the longest dimension and also referred to as both particle size and particle size, can be described more generally using the median of the distribution of the particle sizes, also referred to as the median particle size.

Particle sizes can be determined using various methods, typically light scattering methods including static light scattering (SLS) and dynamic light scattering (DLS), also referred to generally as laser light scattering techniques. In one embodiment, particles of metal detectable agent as disclosed herein have a median particle size (D₅₀), of greater than or equal to 5 micrometers, specifically greater than or equal to 8 micrometers, and more specifically greater than or equal to 10 micrometers. In an alternative embodiment, the particles of metal detectable have a median particle size of less than or equal to 50 micrometers, specifically less than or equal to 20 micrometers. In a specific embodiment, useful metal detectable agents have a median particle size 5 to 25 micrometers, specifically 5 to 10 micrometers. The maximum particle size range (D₉₀, where 90% of the particles are smaller) for Metal detectable agents varies with the median particle size, and can in general be 5 to 50 micrometers, specifically 5 to 25 micrometers, wherein particles having the maximum particle size constitute less than 5 wt%, specifically less than 3 wt%, of the total weight of particles of Metal detectable agent. The distribution of median particle sizes of a metal detectable agent can be unimodal, bimodal, or multimodal.

In an alternative embodiment, particles of metal detectable agent as disclosed herein have a median particle size (D₅₀), of less than or equal to 5 micrometers, specifically less than or equal to 2 micrometers, more specifically less than or equal to 1.5 micrometers, and still more specifically less than or equal to 1 micrometer. In a specific embodiment, useful metal detectable agents have a median particle size from 0.1 to 0.5 micrometers, specifically 0.2 to 0.5 micrometers. In another specific embodiment, useful metal detectable agents have a median particle size 0.5 to 1 micrometer, specifically 0.5 to 0.9 micrometers.

Metal detectable agents can be used in varying stages of compositional purity, provided any impurities present in the thermoplastic composition do not significantly adversely affect the selected properties of the thermoplastic composition. In alternative embodiments, metal detectable agents can have minor amounts of impurities of less than or equal to 0.1% by weight, more specifically less than or equal to 0.01% by weight, and still more specifically less than or equal to 0.001% by weight, based on the total weight of the metal detectable agent. In one embodiment, the metal detectable agent can contain minor amounts of impurities present as the oxide, hydroxide, carboxylate, carbonate, subcarbonates, phosphate, sulfate, sulfonate, silicate, aluminate, or other salt of metals and/or nonmetals such as lithium, sodium, potassium, magnesium, calcium, barium, silicon, iron, zinc, nickel, copper, boron, aluminum, combinations of these, and the like, in minor amounts so that the selected properties of the thermoplastic composition are not significantly adversely affected. In another embodiment, the metal detectable agent may be treated or purified to remove or reduce the presence of such impurities, where such impurities are initially present (prior to treating or purification) in the metal detectable agent in amounts sufficient to significantly adversely affect the selected properties of the composition. The metal detectable agents disclosed herein do not require separation or purification of phases to provide a suitable material.

The metal detectable agents as used can be untreated, or used as treated, coated, and/or dispersed forms. Any suitable surface coating agent, treatment, or dispersant can be used that is suitable to adjust as desired the dispersing properties, adhesion properties, or other such properties of the metal detectable agent particles used herein. In addition, it is contemplated that the metal detectable agent can be in a single structured particle, or as a core-shell structured particle, with the core and shell layers having different phases or compositions. Any such structure is contemplated, provided the inclusion of the structured particle does not have any significantly adverse effects on the properties of the thermoplastic composition.

Specific compounds that are useful as metal detectable agents include magnetite. In one embodiment, the metal detectable agent can include magnetite, as previously discussed. In one embodiment where magnetite is used, the median particle size of the magnetite is greater than 1 micrometers. In a more specific embodiment, the median particle size of magnetite is 5 to 25 micrometers, specifically 5 to 15 micrometers, and still more specifically 5 to 10 micrometers. In another embodiment where magnetite is used, the median particle size of the magnetite is less than 1 micrometer. In a more specific embodiment, the median particle size of magnetite is 0.01 to 0.5 micrometers, specifically 0.1 to 0.5 micrometers, and still more specifically 0.2 to 0.5 micrometers.

The thermoplastic composition may also include mixtures of metal detectable agents. Thus in addition to magnetite, the thermoplastic composition may further include any additional metal detectable agent that does not significantly adversely affect the selected properties of the composition.

In one embodiment, the thermoplastic composition includes a metal detectable agent in an amount of 0.01 to 10 parts by weight, specifically 0.05 to 10 parts by weight, more specifically 0.1 to 10 parts by weight, still more specifically 0.1 to 8 parts by weight, and still yet more specifically 0.1 to 6 parts by weight, based on a combined 100 parts by weight of polysiloxane-polycarbonate and the polycarbonate.

Also in another embodiment, the thermoplastic composition has a siloxane content of 0.1 to 6 wt%, specifically 0.5 to 6 wt%, more specifically 1 to 6 wt%, still more specifically 1 to 5 wt% siloxane units, and still more specifically 1 to 4 wt% siloxane units, based on the total weight of the thermoplastic composition.

The thermoplastic composition thus includes polycarbonate, polysiloxane-polycarbonate polymer, and a metal detectable agent. In one embodiment, polycarbonates specifically useful in the thermoplastic polymer include homopolycarbonates, copolycarbonates, polyester-polycarbonates, blends thereof with polyesters, and combinations including at least one of the foregoing polycarbonate-type resins or blends. In another specific embodiment, a thermoplastic composition consists essentially of polycarbonate, a polysiloxane-polycarbonate polymer, a metal detectable agent, and any additives and/or fillers that are not essential to the composition. In another specific embodiment, the thermoplastic composition consists of polysiloxane-polycarbonate polymer and metal detectable agent. In another specific embodiment, the thermoplastic composition consists of polysiloxane-polycarbonate polymer, metal detectable agent, and an additional polycarbonate.

In addition to the polycarbonate, the polysiloxane-polycarbonate copolymer and metal detectable agent, the thermoplastic composition can further include various other additives ordinarily incorporated with thermoplastic compositions of this type, where the additives are selected so as not to significantly adversely affect the selected properties of the thermoplastic composition. Mixtures of additives may be used. Such additives may be mixed at a suitable time during the mixing of the components for forming the thermoplastic composition.

Useful additives contemplated herein include, but are not limited to, impact modifiers, fillers, colorants including dyes and pigments, antioxidants, heat stabilizers, light and/or UV light stabilizers, plasticizers, lubricants, mold release agents, flame retardants, antistatic agents, anti-drip agents, radiation (gamma) stabilizers, and the like, or a combination including at least one of the foregoing additives. While it is contemplated that other resins and or additives may be used in the thermoplastic compositions described herein, such additives while useful in some exemplary embodiments are not essential.

Surprisingly, it has been found that a thermoplastic composition including polycarbonate, a polysiloxane-polycarbonate and magnetite as the metal detectable agent in amounts of less than 10 wt% of the thermoplastic composition can be used to create a metal detectable polycarbonate-polysiloxane formulation. The particle size of the magnetite is so chosen that key properties (flow, low temperature impact, gloss) of the composition are capable of being maintained. Mechanical properties of the thermoplastic composition are also retained when magnetite having a selected median particle size is used as the metal detectable agent, in particular, to form articles that, when molded from the thermoplastic composition and having a thickness of 3.2 mm, has a notched Izod impact (NII) strength of greater than or equal to 300 J/m, when measured at a temperature of 23°C according to ASTM D256-04, or a notched Izod impact strength of greater than or equal to 150 J/m, when measured at a temperature of 0°C according to ASTM D256-04 (based on 100% of a test set of at least 5 molded samples exhibiting a ductile fracture mode, carried out according to ASTM D256-04).

In embodiments using a metal detectable agent, an article molded from the thermoplastic composition and having a thickness of 3.2 mm has a notched Izod impact (NII) strength of greater than or equal to 350 J/m, specifically greater than or equal to 400 J/m, more specifically greater than or equal to 450 J/m, and still more specifically greater than or equal to 500 J/m, when measured at a temperature of 23°C, according to ASTM D256-04. Also in an embodiment, for a test set of at least 5 molded articles of 3.2 mm thickness and molded from the thermoplastic composition, 100% of the articles exhibited ductile fracture mode when measured at a temperature of 23°C, according to ASTM D256-04.

In alternative embodiments using a metal detectable agent, an article molded from the thermoplastic composition and having a thickness of 3.2 mm has a notched Izod impact (NII) strength of greater than or equal to 200 J/m, specifically greater than or equal to 250 J/m, more specifically greater than or equal to 300 J/m, and still more specifically greater than or equal to 350 J/m, when measured at a temperature of 0°C, according to ASTM D256-04. Also in an embodiment, for a test set of at least 5 molded articles of 3.2 mm thickness and molded from the thermoplastic composition, 100% of the articles exhibited ductile fracture mode when measured at a temperature of 0°C, according to ASTM D256-04.

In those embodiments wherein a metal detectable agent is used, an article molded from the thermoplastic composition demonstrates metal detectability. For example, a metal detector will sound when an article including the thermoplastic composition is scanned against an article including an organic material (such as a chocolate bar), a thermoplastic polymer (such as bisphenol A polycarbonate) or a composition identical to the thermoplastic composition of the instant invention but which does not include the metal detectable agent.

The thermoplastic composition may be manufactured by methods generally available in the art, for example, in one embodiment, in one manner of proceeding, powdered polysiloxane-polycarbonate, the metal detectable agent, and any added polycarbonate, and other additives as desired are first mixed in a HENSCHEL MIXER® high speed mixer. Other low shear processes including but not limited to hand mixing may also accomplish this blending. The blend is then fed into the throat of an extruder via a hopper. Alternatively, one or more of the components may be incorporated into the composition by feeding directly into the extruder at the throat and/or downstream through a sidestuffer. Additives may also be compounded into a masterbatch with a selected polymeric resin and fed into the extruder. In one embodiment, the x-ray conrtrast agent or metal detectable agent is previously compounded with a polysiloxane-polycarbonate masterbatch. The extruder is generally operated at a temperature higher than that necessary to cause the composition to flow, but at which temperature components of the thermoplastic composition do not decompose so as to significantly adversely affect the composition. The extrudate is immediately quenched in a water batch and pelletized. The pellets, so prepared, when cutting the extrudate may be one-fourth inch long or less as desired. Such pellets may be used for subsequent molding, shaping, or forming.

In a metal detectable embodiment, a method of preparing a thermoplastic composition includes melt combining polycarbonate, a polysiloxane-polycarbonate copolymer and a metal detectable agent comprising magnetite. The melt combining can be done by extrusion. In an embodiment, the proportions of polycarbonate, polysiloxane-polycarbonate and metal detectable agent are selected such that the resultant composition maximizes the metal detectable capability while not significantly adversely low temperature NII and ductility.

In a specific embodiment, the extruder is a twin-screw extruder. The extruder is typically operated at a temperature of 180 to 385°C, specifically 200 to 330°C, more specifically 220 to 300°C, wherein the die temperature may be different. The extruded thermoplastic composition is quenched in water and pelletized.

Shaped, formed, or molded articles including the thermoplastic compositions are also provided. The thermoplastic compositions may be molded into useful shaped articles by a variety of means such as injection molding, extrusion, rotational molding, blow molding and thermoforming. In a specific embodiment, molding is done by injection molding. Beneficially, the thermoplastic composition has excellent mold filling capability due to its high flow properties.

The thermoplastic composition can be provided as pellets, and is useful to form articles, including articles for the food service industry such as for example molds, utensils, trays, sheets, packaging, and the like; medical devices and consumables such as bottles, syringes, catheters, labware, and other articles and consumables; and toys including molded plastic toys and packaging. In a specific embodiment, a useful article is a mold for preparing candies, and in a more specific embodiment, the mold is a mold for manufacturing chocolate.

In an embodiment, a mold for manufacturing chocolate and including polysiloxane-polycarbonate, polycarbonate, and the metal detectable agent, exhibits metal detectability, wherein chocolates manufactured with the mold exhibit a high surface gloss and aesthetically pleasing appearance. In another embodiment, the chocolate mold includes 0.01 to 10 parts by weight of a metal detectable agent.

While specific applications and articles are disclosed herein, one skilled in the art will appreciate that the applications of the thermoplastic compositions herein should not be considered as limited to these applications.

The thermoplastic composition is further illustrated by the following non-limiting examples.

All thermoplastic compositions for the examples (abbreviated Ex. in the following tables) and comparative examples (abbreviated Cex. in the following tables) were prepared using one or more of the following components listed in Table 1, to evaluate different inorganic compounds for use as metal detectable agents, and thereby determine the effect of these inorganic compounds on metal detectability of polycarbonate, polycarbonate-polysiloxane compositions.

All thermoplastic compositions were compounded on a Werner and Pfleiderer ZSK 25-mm twin-screw extruder operating at temperatures of from 260 to 300°C. The twin-screw extruder had enough distributive and dispersive mixing elements to produce good mixing of the polymer compositions. The compositions were subsequently dried at 120°C for 4 hours and then molded on a Husky or BOY injection-molding machine using barrel temperatures of from 270 to 300°C and mold temperatures of from 65 to 80°C. It will be recognized by one skilled in the art that the extrusion and molding methods are not limited to these temperatures.

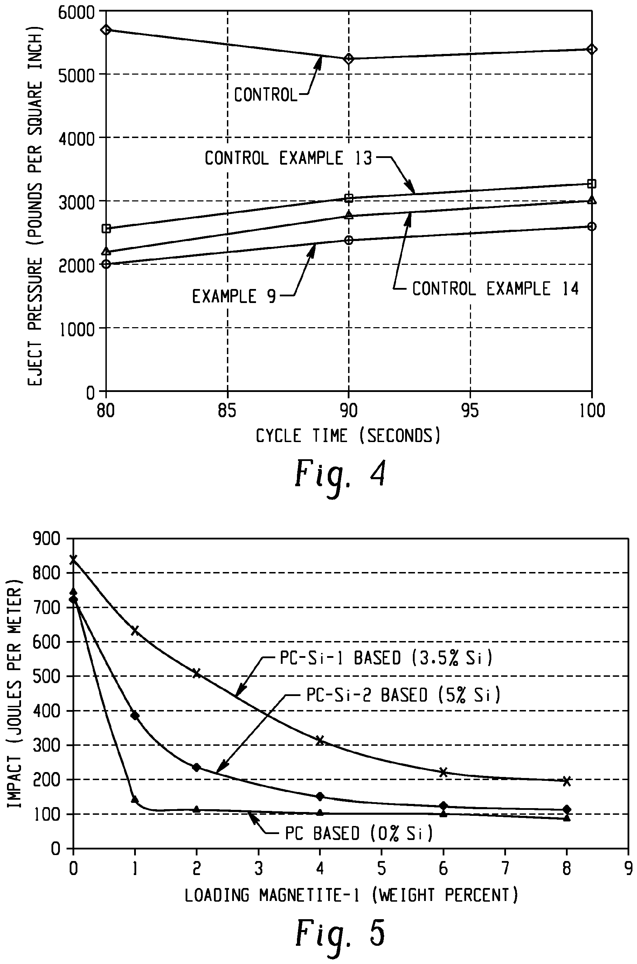

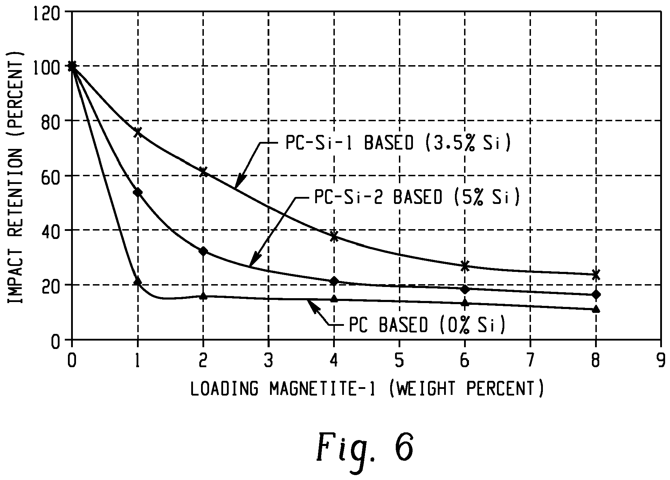

The set of examples and the data were for metal detectable embodiments of the present invention. The components used are set forth below in Table 7 and the corresponding results are set forth in Tables 8 and 9 and FIGS. 5 and 6.

**Table 7. Materials used for metal detectable embodiments**

| Component | Description | Source |
|---|---|---|
| PC-Si-2 | LEXAN™ EXL Polysiloxane-polycarbonate, 20 wt% dimethylsiloxane, average polysiloxane block length of 45 siloxane repeating units, Mw = 30,000 g/mol | SABIC Innovative Plastics |
| PC-Si-3 | LEXAN™ EXL Polysiloxane-polycarbonate, 6 wt% dimethylsiloxane, average polysiloxane block length of 45 siloxane repeating units, Mw = 23,000 g/mol | SABIC Innovative Plastics |
| PC-4 | LEXAN™ Bisphenol A (BPA) Polycarbonate Mw 30,500 g/mol | SABIC Innovative Plastics |
| PC-5 | LEXAN™ Bisphenol A (BPA) Polycarbonate Mw 26,200 g/mol | SABIC Innovative Plastics |
| PC-6 | LEXAN™ Bisphenol A (BPA) Polycarbonate Mw 21,800 g/mol | SABIC Innovative Plastics |
| 1-168 | IRGAFOS® 168 antioxidant (Tris-(2,6-di-tert-butylphenyl)phosphite) | Ciba Specialty Chemicals |
| Magnetite-2 | MAGNIF® 10 functional filler with particle size D₁₀ = 6 µm, D₅₀ = 18 µm (median particle size), and D₉₀ = 41 µm | Minelco, The Netherlands |
| SST | Stainless steel fiber with polycarbonate resin and sizing | Bekaert Fibre Technologies, Belgium |
| Magnetite SMT-01S | Magnetite, median particle size D₅₀ = 0.3 µm | Saehan Media Co., Ltd., Korea |

Notched Izod impact (NII) testing was determined on 3.2 mm thick bars according to ASTM D256-04, at temperatures of 23°C, 0°C, -10°C, -20°C, and -30°C, where the NII impact strength is reported in units of joules per meter (J/m), and the percent ductility is defined as the percent of molded sample articles in a test set of at least 5 samples which exhibit ductile fracture mode.

Polycarbonate-polysiloxane formulations are known and used for their ductile fracture mode at low temperatures. All compositions were tested for Notched Izod impact using standard ASTM D256 parameters from temperatures ranging from 23°C to -30°C. From Table 8, we see that the control formulations show ductile fracture at room temperature with an impact strength of 748 J/m (Cex. 15), 725 J/m (Cex. 23) and 836 J/m. It is beneficial that the metal detectable formulation also be able to retain this property. Table 8 shows Notched Izod impact data for the compositions made with metal detectable fillers. Addition of the filler reduces impact strength significantly at room temperature by half or even more for formulation based on solely polycarbonate (Cex. 16 to Cex. 22) and formulations based on polycarbonate-polysiloxane-2 (Cex. 24 to Cex. 30) with an overall siloxane level of 5 wt%.

Ductile impact is seen at room temperature only and in most cases, fracture mechanism changes to brittle mode, completely or partially depending on the filler at lower temperatures. Addition of the magnetite (fibers or powder) thus significantly reduced the impact strength, even at room temperature, depending on the filler loading as shown in the comparison.

For the polycarbonate formulations (Cex. 16 to Cex.22) there is no formulation that shows ductile impact at 0°C. The average particle size of the fillers used ranged from 5-25 microns; hence effect on impact properties is severe.

Surprisingly it is shown that the combinations of polysiloxane modified PC and Fe3O4 (Ex 11 to Ex 17) are less sensitive for ductility decreases. It is even more surprisingly the these formulations have an overall siloxane level of 3.5%, lower then the overall siloxane level in Cex.24 to Cex. 30).

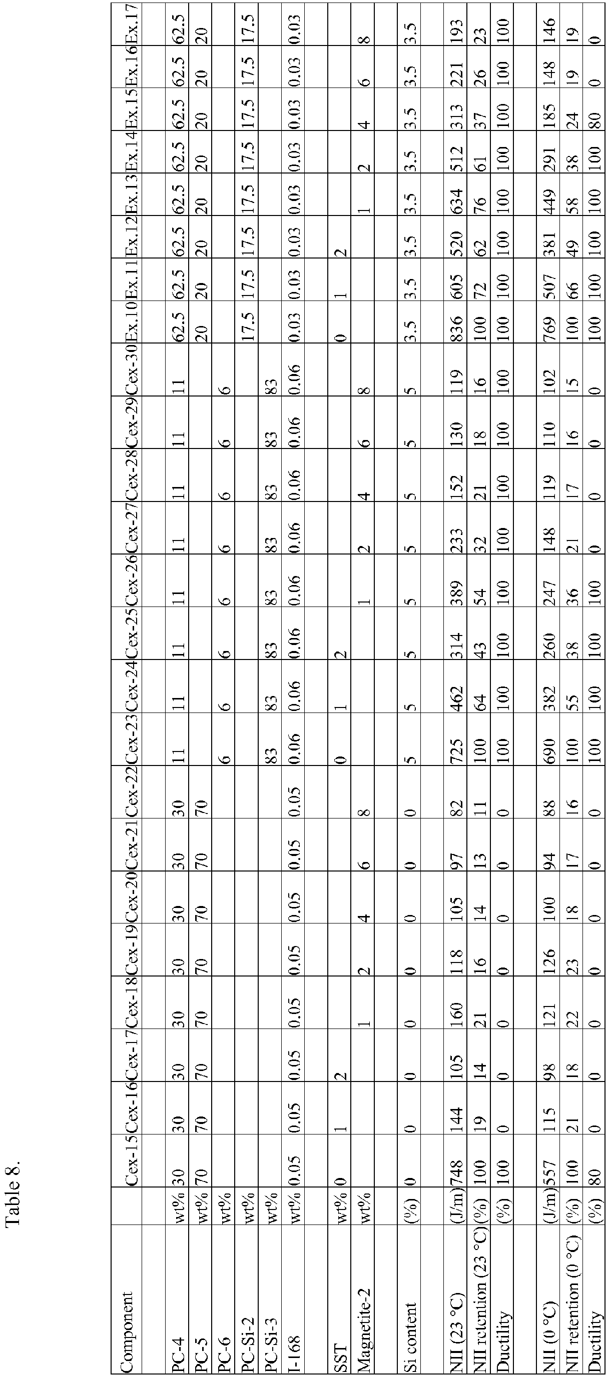

As may be seen, decreasing the particle size of the inorganic additives can lead to better impact retention and other properties, while still maintaining the metal detectablility was also within acceptable limits for each of these examples (i.e., -20% to 0% or greater).

**Table 9.**

| Component | | Cex-31 | Cex-32 | Cex-33 | Cex-34 | Ex.18 | Ex.19 | Ex.20 | Ex.21 |
|---|---|---|---|---|---|---|---|---|---|
| | | | | | | | | | |
| PC-4 | wt% | 30 | 30 | 30 | 30 | 62.5 | 62.5 | 62.5 | 62.5 |
| PC-5 | wt% | 70 | 70 | 70 | 70 | 20 | 20 | 20 | 20 |
| PC-6 | wt% | | | | | | | | |
| PC-Si-2 | wt% | | | | | 17.5 | 17.5 | 17.5 | 17.5 |
| PC-Si-3 | wt% | | | | | | | | |
| I-168 | wt% | 0.05 | 0.05 | 0.05 | 0.05 | 0.03 | 0.03 | 0.03 | 0.03 |
| | | | | | | | | | |
| Magnetite SMT-01S | wt% | 0 | 1 | 2 | 4 | 0 | 1 | 2 | 4 |
| | | | | | | | | | |
| Si content | (%) | 0 | 0 | 0 | 0 | 3.5 | 3.5 | 3.5 | 3.5 |
| | | | | | | | | | |
| NII (23 °C) | (J/m) | 748 | 734 | 742 | 611 | 836 | 819 | 811 | 824 |
| NII (0 °C) | (J/m) | 557 | 598 | 477 | 170 | 769 | 771 | 778 | 779 |
| NII (-20 °C) | (J/m) | 174 | 164 | 252 | 147 | 788 | 741 | 743 | 719 |

As may be seen in Table 9, the Notched izod data for compositions containing Magnetite SMT-01S is provided and it can be seen that the particle size of the magnetite may be adjusted to achieve selected characteristics. In these examples, smaller particle size magnetite was used. In those samples having both the polycarbonate and the polycarbonate-polysiloxane copolymer, the resulting impact strength was much greater than those samples having no polycarbonate-polysiloxane copolymer and these materials also had higher impact strengths than those corresponding samples using larger particle size magnetite (Ex. 13-15). So while larger particle size magnetite helped retain a greater impact strength than stainless steel fiber, if higher impact strength is a selected characteristic, then these examples would indicate that smaller particle size magnetite will help retain a higher percentage of NII than larger particle size magnetite.

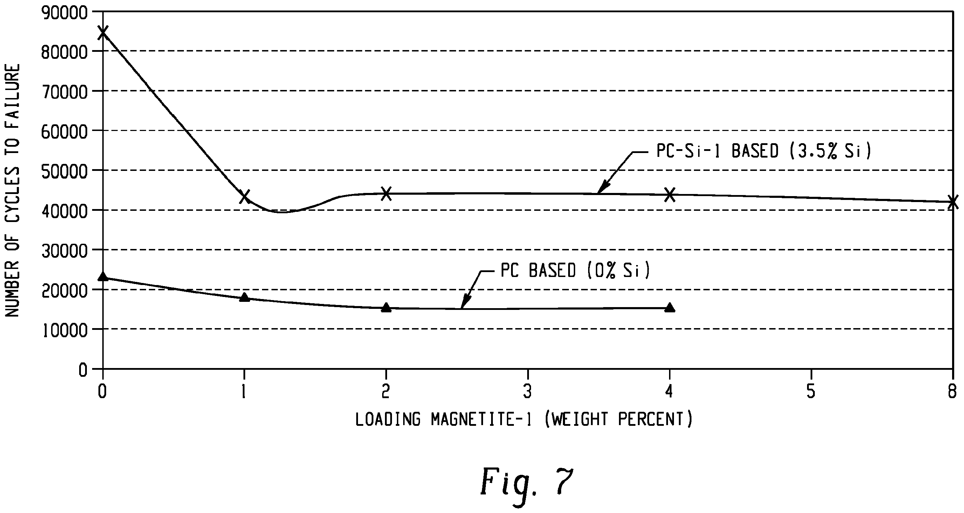

FIGS. 5 and 6 also help show that polysiloxane based polycarbonate and Fe3O4 show good toughness, and toughness retention at low temperature. The data shown relates to 0, 3.5 and 5 total wt.% Si. In line with these results, as expected and shown in FIG. 7 and Table 10, fatigue (which is a beneficial property for chocolate molds) for PC Si-based system was significantly better compared to PC-based system (0% total wt.% Si) in the presence of magnetite.

**Table 10.**

| | Sample no. | Stress, Mpa | No of cycles to failure | aver | std |
|---|---|---|---|---|---|
| PC-4 | 1 | 30 | 19171 | 22703.5 | 4995.709 |
| | 2 | 30 | 26236 | | |
| PC-4 + 1% Magnetite-2 | 1 | 30 | 18795 | 17308.5 | 2102.228 |
| | 2 | 30 | 15822 | | |
| PC-4 + 2% Magnetite-2 | 1 | 30 | 16112 | 15127 | 1393 |
| | 2 | 30 | 14142 | | |
| PC-4 + 4% Magnetite-2 | 1 | 30 | 13308 | 14483 | 1661.701 |
| | 2 | 30 | 15658 | | |
| PC-Si-2 | 1 | 30 | 85544 | 84379.5 | 1646.852 |
| | 2 | 30 | 83215 | | |
| PC-Si-2 +1% Magnetite-2 | 1 | 30 | 43445 | 42738 | 999.849 |
| | 2 | 30 | 42031 | | |
| PC-Si-2 +2% Magnetite-2 | 1 | 30 | 44891 | 44108 | 1107.329 |
| | 2 | 30 | 43325 | | |
| PC-Si-2 +4% Magnetite-2 | 1 | 30 | 46537 | 43638 | 4099.805 |
| | 2 | 30 | 40739 | | |
| PC-Si-2 +6% Magnetite-2 | 1 | 30 | 41818 | 42436.5 | 874.6911 |
| | 2 | 30 | 43055 | | |

Thus the above Examples and Comparative Examples demonstrate that metal detectable additives can enhance the properties of polysiloxane-polycarbonate copolymer-containing compositions without compromising properties such as mold release, melt flow, low temperature ductility, or gloss.

In another embodiment, an article comprising a thermoplastic composition comprises a) from 65 to 95% by weight of polycarbonate b) from 5 to 35% by weight of a polysiloxane-polycarbonate copolymer; and c) from 0.01 to 10% by weight of a metal detectable agent, comprising magnetite, wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04, wherein the polysiloxane-polycarbonate copolymer comprises 65 to 85 wt% of carbonate units and 5-35 wt% siloxane units, based on the total weight of the polysiloxane-polycarbonate.

In yet another embodiment, an article comprising a thermoplastic composition comprises a) from 65 to 95% by weight of polycarbonate b) from 5 to 35% by weight of a polysiloxane-polycarbonate copolymer; and c) from 0.01 to 10% by weight of a metal detectable agent, comprising magnetite, wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04, wherein the polysiloxane-polycarbonate copolymer comprises 65 to 85 wt% of carbonate units and 5-35 wt% siloxane units, based on the total weight of the polysiloxane-polycarbonate, wherein the article comprises polysiloxane-polycarbonate in an amount of 10 to 25 parts by weight, and polycarbonate in an amount of 75 to 90 parts by weight, based on combined 100 parts by weight of polysiloxane-polycarbonate and any added polycarbonate and wherein the metal detectable agent has a median particle size of less than 50 micrometers.

In one embodiment, an article comprising a thermoplastic composition comprises a) from 65 to 95% by weight of polycarbonate b) from 5 to 35% by weight of a polysiloxane-polycarbonate copolymer; and c) from 0.01 to 10% by weight of a metal detectable agent, comprising magnetite, wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04, wherein the polysiloxane-polycarbonate copolymer comprises 65 to 85 wt% of carbonate units and 5-35 wt% siloxane units, based on the total weight of the polysiloxane-polycarbonate, the article being an article for the food service industry, a medical device, or a toy.

In one embodiment, a method for increasing the metal detectability in an article comprising a thermoplastic composition, comprises combining a metal detectable agent with polycarbonate and a polysiloxane-polycarbonate copolymer, the metal detectable agent comprising magnetite; and forming the article from the thermoplastic composition, wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04.

In one embodiment, a mold for manufacturing a food product, comprising a thermoplastic composition comprises a) from 65 to 95% by weight of polycarbonate b) from 5 to 35% by weight of a polysiloxane-polycarbonate copolymer; and c) from 0.01 to 10% by weight of a metal detectable agent, comprising magnetite, wherein an article molded from the thermoplastic composition and having a thickness of 3.2 mm has a notched Izod impact (NII) strength of greater than or equal to 200 J/m, when measured at a temperature of 23°C according to ASTM D256-04, or a notched Izod impact strength of greater than or equal to 125 J/m, when measured at a temperature of 0°C according to ASTM D256-04.

All ranges disclosed herein are inclusive of the endpoints, and the endpoints are independently combinable with each other.

The use of the terms "a" and "an" and "the" and similar referents in the context of describing the invention (especially in the context of the following claims) are to be construed to cover both the singular and the plural, unless otherwise indicated herein or clearly contradicted by context. Further, it should further be noted that the terms "first," "second," and the like herein do not denote any order, quantity, or importance, but rather are used to distinguish one element from another.

## Claims

1. An article comprising a thermoplastic composition comprising:
a) from 65 to 95% by weight of polycarbonate
b) from 5 to 35% by weight of a polysiloxane-polycarbonate copolymer; and
c) from 0.01 to 10% by weight of a metal detectable agent, comprising magnetite,
wherein an article molded from the thermoplastic composition and having a thickness of 3.2 mm has a notched Izod impact (NII) strength of greater than or equal to 200 J/m, when measured at a temperature of 23ºC according to ASTM D256-04, or a notched Izod impact strength of greater than or equal to 125 J/m, when measured at a temperature of 0ºC according to ASTM D256-04.

2. The article of Claim 1, wherein the polysiloxane-polycarbonate copolymer comprises 65 to 85 wt% of carbonate units and 5-35 wt% siloxane units, based on the total weight of the polysiloxane-polycarbonate.

3. The article of any one of Claims 1 or 2, comprising polysiloxane-polycarbonate copolymer in an amount of 10 to 25 parts by weight, and polycarbonate in an amount of 75 to 90 parts by weight, based on combined 100 parts by weight of polysiloxane-polycarbonate and any added polycarbonate.

4. The article of Claim 1, wherein the metal detectable agent has a median particle size of less than 50 micrometers.

5. The article of Claim 1, wherein the metal detectable agent has a median particle size of less than or equal to 1 micrometer.

6. The article of Claim 1, wherein the thermoplastic composition further comprises additives including impact modifiers, fillers, colorants including dyes and pigments, antioxidants, heat stabilizers, light, stabilizers, UV light stabilizers, plasticizers, lubricants, mold release agents, flame retardants, antistatic agents, anti-drip agents, radiation stabilizers, or a combination comprising at least one of the foregoing additives.

7. The article of Claim 1, wherein the article is an article for the food service industry, a medical device, or a toy.

8. A method for increasing the metal detectability in an article comprising a thermoplastic composition, comprising
combining
a metal detectable agent, with polycarbonate and a polysiloxane-polycarbonate copolymer; the metal detectable agent comprising magnetite ,
and
forming the article from the thermoplastic composition,
wherein an article molded from the thermoplastic composition and having a thickness of 3.2 mm has a notched Izod impact (NII) strength of greater than or equal to 200 J/m, when measured at a temperature of 23ºC according to ASTM D256-04, or a notched Izod impact strength of greater than or equal to 125 J/m, when measured at a temperature of 0ºC according to ASTM D256-04.

9. The article of claim 1, wherein the article is a mold for manufacturing a food product.

## Patentansprüche

1. Artikel, der eine thermoplastische Zusammensetzung umfasst, umfassend:
a) von 65 bis 95 Gew.-% Polycarbonat
b) von 5 bis 35 Gew.-% eines Polysiloxan-Polycarbonat-Copolymers; und
c) von 0,01 bis 10 Gew.-% eines metalldetektierbaren Mittels, das Magnetit umfasst,
wobei ein Artikel, der aus der thermoplastischen Zusammensetzung geformt ist und eine Dicke von 3,2 mm aufweist, eine Izod-Kerbschlagzähigkeit (NII-Zähigkeit) größer gleich 200 J/m, wenn bei einer Temperatur von 23 °C gemäß ASTM D256-04 gemessen wird, oder eine Izod-Kerbschlagzähigkeit größer gleich 125 J/m, wenn bei einer Temperatur von 0 °C gemäß ASTM D256-04 gemessen wird, aufweist.

2. Artikel nach Anspruch 1, wobei das Polysiloxan-Polycarbonat-Copolymer 65 bis 85 Gew.-% Carbonat-Einheiten und 5-35 Gew.-% SiloxanEinheiten, bezogen auf das Gesamtgewicht des Polysiloxan-Polycarbonats, umfasst.

3. Artikel nach einem der Ansprüche 1 oder 2, der Polysiloxan-Polycarbonat-Copolymer in einer Menge von 10 bis 25 Gewichtsteilen und Polycarbonat in einer Menge von 75 bis 90 Gewichtsteilen, bezogen auf kombinierte 100 Gewichtsteile von Polysiloxan-Polycarbonat und etwaigem zugegebenen Polycarbonat, umfasst.

4. Artikel nach Anspruch 1, wobei das metalldetektierbare Mittel eine mittlere Teilchengröße von weniger als 50 Mikrometer aufweist.

5. Artikel nach Anspruch 1, wobei das metalldetektierbare Mittel eine mittlere Teilchengröße kleiner gleich 1 Mikrometer aufweist.

6. Artikel nach Anspruch 1, wobei die thermoplastische Zusammensetzung weiterhin Additive, einschließlich Schlagzähigkeitsverbesserern, Füllstoffen, Farbmitteln, einschließlich Farbstoffen und Pigmenten, Antioxidationsmitteln, Wärmestabilisatoren, Lichtstabilisatoren, UV-Lichtstabilisatoren, Weichmachern, Gleitmitteln, Formentrennmitteln, Flammschutzmitteln, Antistatika, Antitropfmitteln, Strahlenschutzmitteln, oder eine Kombination, die mindestens eines der vorstehenden Additive umfasst, umfasst.

7. Artikel nach Anspruch 1, wobei der Artikel ein Artikel für die Lebensmitteldienstleistungsbranche, eine medizinische Vorrichtung oder ein Spielzeug ist.

8. Verfahren zur Erhöhung der Metalldetektierbarkeit in einem Artikel, der eine thermoplastische Zusammensetzung umfasst, umfassend
Kombinieren
eines metalldetektierbaren Mittels mit Polycarbonat und einem Polysiloxan-Polycarbonat-Copolymer; wobei das metalldetektierbare Mittel Magnetit umfasst,
und
Bilden des Artikels aus der thermoplastischen Zusammensetzung,
wobei ein Artikel, der aus der thermoplastischen Zusammensetzung geformt ist und eine Dicke von 3,2 mm aufweist, eine Izod-Kerbschlagzähigkeit (NII-Zähigkeit) größer gleich 200 J/m, wenn bei einer Temperatur von 23 °C gemäß ASTM D256-04 gemessen wird, oder eine Izod-Kerbschlagzähigkeit größer gleich 125 J/m, wenn bei einer Temperatur von 0 °C gemäß ASTM D256-04 gemessen wird, aufweist.

9. Artikel nach Anspruch 1, wobei der Artikel eine Form zur Herstellung eines Lebensmittelprodukts ist.

## Revendications

1. Article comprenant une composition thermoplastique comprenant :
a) 65 à 95% en poids de polycarbonate
b) 5 à 35% en poids d'un copolymère de polysiloxane-polycarbonate ; et
c) 0,01 à 10% en poids d'un agent détectable de métaux, comprenant de la magnétite,
dans lequel un article moulé à partir de la composition thermoplastique et ayant une épaisseur de 3,2 mm a une résistance au choc Izod sur barreau entaillé (NII) supérieure ou égale à 200 J/m, lorsqu'elle est mesurée à une température de 23°C selon la norme ASTM D256-04, ou une résistance au choc Izod sur barreau entaillé supérieure ou égale à 125 J/m, lorsqu'elle est mesurée à une température de 0°C selon la norme ASTM D256-04.

2. Article de la revendication 1, dans lequel le copolymère de polysiloxane-polycarbonate comprend 65 à 85% en poids d'unités carbonate et 5 à 35% en poids d'unités siloxane, par rapport au poids total du polysiloxane-polycarbonate.

3. Article de l'une quelconque des revendications 1 et 2, comprenant un copolymère de polysiloxane-polycarbonate en une quantité allant de 10 à 25 parties en poids et du polycarbonate en une quantité allant de 75 à 90 parties en poids, par rapport à 100 parties en poids combinées de polysiloxane-polycarbonate et de tout polycarbonate ajouté.

4. Article de la revendication 1, dans lequel l'agent détectable de métaux a une taille moyenne de particules inférieure à 50 micromètres.

5. Article de la revendication 1, dans lequel l'agent détectable de métaux a une taille moyenne de particules inférieure ou égale à 1 micromètre.

6. Article de la revendication 1, dans lequel la composition thermoplastique comprend en outre des additifs comportant des modificateurs de résistance aux chocs, des charges, des colorants comportant des matières colorantes et des pigments, des antioxydants, des stabilisants thermiques, des photostabilisants, des stabilisants anti-UV, des plastifiants, des lubrifiants, des agents de démoulage, des agents ignifuges, des agents antistatiques, des agents anti-gouttes, des stabilisants de rayonnements ou une combinaison comprenant au moins l'un des additifs précédents.

7. Article de la revendication 1, dans lequel l'article est un article pour l'industrie de la restauration, un dispositif médical ou un jouet.

8. Procédé pour augmenter la détectabilité de métaux dans un article comprenant une composition thermoplastique, comprenant le fait
de combiner
un agent détectable de métaux avec du polycarbonate et un copolymère de polysiloxane-polycarbonate ; l'agent détectable de métaux comprenant de la magnétite,
et
de former l'article à partir de la composition thermoplastique,
dans lequel un article moulé à partir de la composition thermoplastique et ayant une épaisseur de 3,2 mm a une résistance au choc Izod sur barreau entaillé (NII) supérieure ou égale à 200 J/m, lorsqu'elle est mesurée à une température de 23°C selon la norme ASTM D256-04, ou une résistance au choc Izod sur barreau entaillé supérieure ou égale à 125 J/m, lorsqu'elle est mesurée à une température de 0°C selon la norme ASTM D256-04.

9. Article de la revendication 1, dans lequel l'article est un moule pour la fabrication d'un produit alimentaire.
